Europäisches Patentamt

(19) European Patent Office

Office européen des brevets

(11) Publication number: **0 138 526**
**B1**

(12) **EUROPEAN PATENT SPECIFICATION**

(45) Date of publication of patent specification: **26.04.89**

(21) Application number: **84306803.2**

(22) Date of filing: **05.10.84**

(51) Int. Cl.⁴: **C 12 P 13/22**

(54) Method of producing L-phenylalanine by fermentation, and bacteria therefor.

(30) Priority: **07.10.83 JP 188041/83**

(43) Date of publication of application:
**24.04.85 Bulletin 85/17**

(45) Publication of the grant of the patent:
**26.04.89 Bulletin 89/17**

(84) Designated Contracting States:
**DE FR IT NL**

(56) References cited:
**FR-A-2 059 043**
**US-A-3 909 353**
**CHEMICAL ABSTRACTS, vol. 80, no. 5, 4th Febr 1974, p. 318, no. 25919w, Columbus, Ohio (US)**
**CHEMICAL ABSTRACTS, vol. 84, no. 7, 16th Febr 1976, p. 328, no. 41979d, Columbus, Ohio (US)**
**CHEMICAL ABSTRACTS, vol. 100, no. 11, 12th March 1984, p. 430, no. 84237b, Columbus, Ohio (US)**
**AGRICULTURAL AND BIOLOGICAL CHEMISTRY, vol. 45, no. 10, October 1981, Tokyo JP); I.SHIIO et al.: "Regulation at metabolic branch points of aromatic amino acid biosynthesis in brevibacterium flavum", pp. 2197-2207**

(73) Proprietor: **AJINOMOTO CO., INC.**
**5-8, Kyobashi 1-chome, Chuo-ku**
**Tokyo 104 (JP)**

(72) Inventor: **Tsuchida, Takayasu**
**No. 1730-13, Kamikurata-cho Totsuka-ku**
**Yokohama-shi Kanagawa-ken (JP)**
Inventor: **Kurahashi, Osamu**
**No. 958, Kashimada Saiwai-ku**
**Kawasaki-shi Kanagawa-ken (JP)**
Inventor: **Kawashima, Hiroki**
**No. 2-20-8, Kannon Kawasaki-ku**
**Kanagawa-ken (JP)**
Inventor: **Enei, Hitoshi**
**No. 2-30-2, Ikego**
**Zushi-shi Kanagawa-ken (JP)**

(74) Representative: **Armitage, Ian Michael et al**
**MEWBURN ELLIS & CO. 2/3 Cursitor Street**
**London EC4A 1BQ (GB)**

(56) References cited:
**CHEMICAL ABSTRACTS, vol. 101, no. 13, 24th Sept 1984, p. 502, no. 108948h, Columbus, Ohio (US)**

Courier Press, Leamington Spa, England.

# EP 0 138 526 B1

**Description**

The present invention relates to a method of producing L-phenylalanine (hereinafter simply referred to as phenylalanine) by a fermentation process.

Heretofore, there has been known, as a method of producing phenylalanine, a method involving the use of tyrosine auxotrophy of bacterial belonging to the genus Brevibacterium or the genus Micrococcus (Japanese Published Examined Patent Application 6345/62), a method involving the use of a mutant requiring tyrosine for the growth thereof and having resistance to 5-methyltryptophane, a method involving the use of mutants having resistance to phenylalanine analogues (Japanese Published Examined Patent Application 28712/76), a method involving the use of a decoinine-sensitive mutant (Japanese Published Examined Patent Application 64793/71), etc.

On the other hand, with respect to the genus Bacillus, a method involving the use of an L-tyrosine auxotrophic mutant (Japanese Published Examined Patent Application 6345/62), a method involving the use of a mutant having tryptophane auxotrophy and resistance to p-fluorophenylalanine and to β-2-thienyl-alanine (Japanese Published Unexamined Patent Application 67488/73), etc. are known. However, amounts of phenylalanine accumulated are merely about 0.2—0.4 g/dl in these methods and much inferior to the phenylalanine-producing bacteria belonging to the genus Brevibacterium or the genus Corynebacterium described above. For this reason, growth of strains has been scarcely investigated for the purpose of industrial production of phenylalanine. In fact, no bacteria having high productivity of phenylalanine is known.

The present inventors have conducted the growth of phenylalanine-producing bacteria for the purpose of developing a method of industrially producing phenylalanine by microorganisms belonging to the genus Bacillus and, as result, have discovered that by imparting auxotrophy for both trypophane and tyrosine as well as resistance to m-fluorophenylalanine (mFP) and/or cinnamic acid, a strain which produces and accumulates phenylalanine in substantial amounts can be obtained.

These mutants also show resistance to compounds chemically analogous to phenylalanine. Specific examples of such phenylalanine analogues include, β-amino-β-phenylpropionic acid, o-fluorophenylalanine, p-fluorophenylalanine, β-2-thienylalanine, β-3-thienylalanine, β-2-furylalanine, β-3-furylalanine, o-aminophenylalanine, p-aminophenylalanine, m-aminophenylalanine, α-amino-β-phenylethanesulfonate, β-2-pyrrolalanine, 1-cyclopentene-1-alanine, 1-cyclohexene-1-alanine, β-4-pyridyl-alanine, β-4-pyrazolylalanine, p-nitrophenylalanine, β-4-thiazolealanine, cyclohexylalanine, 2-amino-4-methyl-4-hexenoic acid, S-(1,2-dichlorovinyl)cysteine, o-chlorophenylalanine, m-chlorophenylalanine, p-chlorophenylalanine, o-bromophenylalanine, m-bromophenylalanine, p-bromophenylalanine, m-fluorotyrosine, 3-nitrotyrosine, β-phenylserine and 3-iodotyrosine.

The present invention has been accomplished based on this discovery.

Microorganisms which may be used in the present invention include the following mutants:

Bacillus subtilis AJ 12095
   FERM P-7286 FERM BP-607 (Trp⁻, Tyr⁻, mFPʳ)

Bacillus subtilis AJ 12096
   FERM P-7287 FERM BP-608 (Trp⁻, Tyr⁻, cinʳ)

Bacillus subtilis AJ 12097
   FERM P-7288 FERM BP-609 (Trp⁻, Tyr⁻, mFPʳ, cinʳ)

Trp⁻: tryptophane auxotrophy
Tyr⁻: tyrosine auxotrophy
mFPLʳ: resistance to m-fluorophenylalanine
cinʳ: resistance to cinnamic acid.

The mutants identified above by FERM P number were originally deposited on October 1, 1983 at the Fermentation Research Institute, Agency of Industrial Sciences and Technology, Ministry of International Trade and Industry (FRI), 1—3, Migashi 1-chome, Yataba-machi, Tsukuba-gun, Ibaragi 305, Japan; and have been accorded the FERMP number indicated above. The mutant deposits were then converted into deposits under the Budapest Treaty on September 17, 1984, and have been accorded the corresponding FERM BP numbers.

The mutants employed in the present invention may be obtained by using a wild strain belonging to the genus Bacillus or known phenylalanine-producing bacteria as a parent strain, and subjecting it to conventional operations to induce mutation, for example, by irradiation with ultraviolet rays or x-rays, or treatment with a chemical such as N-methyl-N'-nitro-N-nitrosoguanidine (referred to as NG), nitrous acid, etc.


## Experiment 1

Phenylalanine-producing bacterium AJ 12094 (FERM P-7285) having auxotrophy for both trypotophane and tyrosine, which had been induced from Bacillus subtilis AJ 11708, was cultured in yeast-bouillon agar slant medium. The grown bacterium was collected and suspended in 1/50M phosphate buffer (pH 7.0; containing 16⁹/ml of cells). To the suspension NG (NG concentration, 200 µg/ml) was added followed by maintaining room temperature for 30 minutes.

After thoroughly washing the NG-treated cells with the same phosphate buffer, the cells were

2

inoculated on minimum agar plate medium containing 1000 µg/ml of mFP shown in Table 1 and cultured at 31.5°C for 7 days.

Table 1. Composition of Minimum Medium

(pH 7.0)

| Component | Concentration |
|---|---|
| Glucose | 20 g/l |
| Ammonium sulfate | 5 g/l |
| Urea | 2 g/l |
| $KH_2PO_4$ | 1 g/l |
| $MgSO_4 \cdot 7H_2O$ | 1 g/l |
| $Fe^{++}$, $Mn^{++}$ ions | 2 ppm each |
| Biotin | 50 µg/l |
| Thiamine hydrochloride | 200 µg/l |
| L-Tryptophane | 150 mg/l |
| L-Tyrosine | 150 mg/l |

Among colonies grown on the agar plate, big colonies were harvested as mFP-resistant strains. Many of the thus obtained resistant strains were found to have superior phenylalanine productivity to the parent strain, from which strain AJ 12095 (FERM BP-607) having the highest productivity was chosen. By similar mutating operations, AJ 12096 (FERM BP-608) was induced by imparting cinnamic acid resistance to AJ 12094 and AJ 12097 (FERM BP-609) by imparting cinnamic acid resistance to AJ 12095 (FERM BP-607).

Experiment 2

In test tubes, 4.0 ml each of the minimum medium (Table 1) containing mFP or cin in such concentrations as shown in Table 2 was separately charged followed by sterilization by heating. A determined amount of the above-mentioned mutant strains was inoculated on the medium and subjected to shake culture at 31.5°C for 24 hours. Each of the culture solutions was diluted with water by 26 times and the absorbancy at 562 nm was measured to determine the degree of growth. The results are shown in Table 2. In Table 2, a relative growth value is shown when the degree of growth without adding any chemical is made 100.

### Table 2. Resistance to Chemical

| Strain | Chemical (µg/ml) | | | | | | | |
|---|---|---|---|---|---|---|---|---|
| | mFP | | | | cin | | | |
| | 0 | 500 | 1000 | 2000 | 0 | 200 | 500 | 1000 |
| AJ 12094 | 100 | 40 | 0 | 0 | 100 | 25 | 0 | 0 |
| AJ 12095 (FERM BP-607) | 100 | 100 | 100 | 90 | 100 | 30 | 0 | 0 |
| AJ 12096 (FERM BP-608) | 100 | 45 | 0 | 0 | 100 | 100 | 55 | 10 |
| AJ 12097 (FERM BP-609) | 100 | 100 | 100 | 95 | 100 | 100 | 95 | 50 |

The culture medium employed in the present invention is a conventional nutrient medium containing a carbon source, a nitrogen source, inorganic salts, L-tyrosine, L-tryptophane, and, if necessary, organic minor nutrients, such as other amino acids, vitamins, nucleic acids, etc. As the carbon source, it may be sufficient as long as the mutant used can utilize it, for example, sugars such as glucose, fructose, sucrose, maltose, hydrolysates of starch, molasses, etc. are employed. Further, alcohols such as ethanol, propanol, etc., organic acids such as acetic acid, citric acid, etc. are employed.

As the nitrogen source, ammonium salts such as ammonium sulfate, ammonium chloride, ammonium phosphate, etc., inorganic or organic nitrogen source such as nitrates, urea, ammonia, meat extract, etc. are employed. As the organic minor nutrients, amino acids, vitamins, fatty acids and nucleic acids, and further, peptone, Casamino acid, yeast extract and protein decomposates, etc. containing amino acids, vitamins, fatty acids and nucleic acids, are employed.

With mutants requiring amino acids such as L-tyrosine, L-tryptophane, etc., it is necessary to supplement nutrients, etc. required.

It is desired that the incubation be performed under aerobic conditions. By performing shake culture or aerial agitation culture for 1 to 4 days while controlling the pH of the medium to 5 to 9 and the temperature at 20 to 40°C during the incubation, phenylalanine is accumulated in the culture solution in marked amounts. Harvesting phenylalanine from the culture solution may be in accordance with known methods; after separating the bacteria from the culture solution phenylalanine is harvested by concentration and crystallization, use of ion exchange resins, etc.

Hereafter the present invention will be explained with reference to the example below.

### Example 1

A medium for producing phenylalanine shown in Table 3 described below was prepared. 500 ml shake flasks were each charged with 20 ml of the medium and sterilized by heating at 120°C for 10 minutes. Then 10 g of calcium carbonate powder, separately sterilized by heating, was added thereto.

Table 3.  Composition of Phenylalanine-Producing Medium

| Component | Content in 1.0 Liter |
|---|---|
| Glucose | 80.0 g |
| Ammonium sulfate | 10.0 g |
| KCl | 2.0 g |
| $KH_2PO_4$ | 1.0 g |
| $MgSO_4 \cdot 7H_2O$ | 0.4 g |
| $Fe^{++}$, $Mn^{++}$ | 2 ppm each |
| L-Tyrosine | 0.15 g |
| L-Tryptophane | 0.20 g |
| Soybean protein decomposate* | 20  ml |
| (* T-N concentration:  4 g/dl) | |

In each sample, a platinum loop amount of one of the phenylalanine-producing bacteria shown in Table 4 was inoculated and subjected to shake culture at 30°C for 72 hours. The amount of phenylalanine produced in each culture solution was measured. The results are shown in Table 4.

Table 4.   Amount of L-Phenylalanine Accumulated

| Strain | Property | L-Phenylalanine (mg/ml) |
|---|---|---|
| AJ 12094 | $Trp^-$, $Tyr^-$ | 2.1 |
| AJ 12095 (FERM BP-607) | $Trp^-$, $Tyr^-$, $mFP^r$ | 7.6 |
| AJ 12096 (FERM BP-608) | $Trp^-$, $Tyr^-$, $cin^r$ | 7.5 |
| AJ 12097 (FERM BP-609) | $Trp^-$, $Tyr^-$, $mFP^r$, $cin^r$ | 10.0 |

**Claims**

1. A method of producing L-phenylalanine which comprises culturing in a liquid medium an L-phenylalanine-producing bacterium belonging to the genus Bacillus, showing auxotrophy for both tryptophane and tyrosine and having resistance to m-fluorophenylalanine and/or cinnamic acid, and recovery L-phenylalanine which has been produced in said liquid medium.
2. A method as set forth in claim 1, wherein said bacterium is a strain of Bacillus subtilis.
3. A method as set forth in claim 2, wherein said bacterium is Bacillus subtilis FERM BP-607.
4. A method as set forth in claim 2, wherein said bacterium is Bacillus subtilis FERM BP-608.
5. A method as set forth in claim 2, wherein said bacterium is Bacillus subtilis FERM BP-609.
6. An L-phenylalanine-producing bacterium belonging to the genus Bacillus, showing auxotrophy for both tryptophane and tyrosine and having resistance to m-fluorophenylalanine and/or cinnamic acid.
7. A bacterium of claim 6 wherein said bacterium is a strain of Bacillus subtilis.
8. A bacterium of claim 7 wherein said bacterium is Bacillus subtilis FERM BP-607.
9. A bacterium of claim 7 wherein said bacterium is Bacillus subtilis FERM BP-608.
10. A bacterium of claim 7 wherein said bacterium is Bacillus subtilis FERM BP-609.

**Patentansprüche**

1. Verfahren zur Herstellung von L-Phenylalanin, bei dem in einem flüssigen Medium ein L-Phenylalanin bildendes Bakterium des Genus Bacillus gezüchtet wird, welches Auxotrophie sowohl für Tryptophan, als aus für Tyrosin zeigt und Resistenz gegen m-Fluorphenylalanin und/oder Zimtsäure hat, und das in dem flüssigen Medium gebildete L-Phenylalanin gewonnen wird.

2. Verfahren gemäß Anspruch 1, bei dem das Bakterium ein Stamm von Bacillus subtilis ist.

3. Verfahren gemäß Anspruch 2, bei dem das Bakterium Bacillus subtilis FERM BP-607 ist.

4. Verfahren gemäß Anspruch 2, bei dem das Bakterium Bacillus subtilis FERM BP-608 ist.

5. Verfahren gemäß Anspruch 2, bei dem das Bakterium Bacillus subtilis FERM BP-609 ist.

6. L-Phenylalanin bildendes Bakterium des Genus Bacillus, welches Auxotrophie sowohl für Tryptophan, als auch für Tyrosin zeigt und Resistenz gegen m-Fluorphenylalanin und/oder Zimtsäure hat.

7. Bakterium gemäß Anspruch 6, wobei das Bakterium ein Stamm von Bacillus subtilis ist.

8. Bakterium gemäß Anspruch 7, wobei das Bakterium Bacillus subtilis FERM BP-607 ist.

9. Bakterium gemäß Anspruch 7, wobei das Bakterium Bacillus subtilis FERM BP-608 ist.

10. Bakterium gemäß Anspruch 7, wobei das Bakterium Bacillus subtilis FERM BP-609 ist.

**Revendications**

1. Un procédé de production de la L-phénylalanine qui comprend la culture dans un milieu liquide d'une bactérie productrice de L-phénylalanine appartenant au genre Bacillus, montrant une auxotrophie vis-à-vis à la fois du tryptophane et de la tyrosine et ayant une résistance à la m-fluorophénylalanine et/ou à l'acide cinnamique, et la récupération de la L-phénylalanine qui a été produite dans ledit milieu liquide.

2. Un procédé selon la revendication 1, selon lequel ladite bactérie est une souche de Bacillus subtilis.

3. Un procédé selon la revendication 2, selon lequel ladite bactérie est le Bacillus subtilis FERM BP-607.

4. Un procédé selon la revendication 2, selon lequel ladite bactérie est le Bacillus subtilis FERM BP-608.

5. Un procédé selon la revendication 2, selon lequel ladite bactérie est le Bacillus subtilis FERM BP-609.

6. Une bactérie productrice de L-phénylalanine appartenant au genre Bacillus, montrant une auxotrophie vis-à-vis à la fois du tryptophane et de la tyrosine et ayant une résistance à la m-fluorophénylalanine et/ou à l'acide cinnamique.

7. Une bactérie selon la revendication 6, selon laquelle cette bactérie est une souche de Bacillus subtilis.

8. Une bactérie selon la revendication 7, selon laquelle ladite bactérie est le Bacillus subtilis FERM BP-607.

9. Une bactérie selon la revendication 7, selon laquelle ladite bactérie est le Bacillus subtilis FERM BP-608.

10. Une bactérie selon la revendication 7, selon laquelle ladite bactérie est le Bacillus subtilis FERM BP-609.